Europäisches Patentamt

European Patent Office

Office européen des brevets

(11) Veröffentlichungsnummer: **0 463 466 A2**

(12) **EUROPÄISCHE PATENTANMELDUNG**

(21) Anmeldenummer: **91109574.3**

(22) Anmeldetag: **11.06.91**

(51) Int. Cl.5: **C07C 227/04**, C07C 229/64

(30) Priorität: **23.06.90 DE 4020056**

(43) Veröffentlichungstag der Anmeldung:
**02.01.92 Patentblatt 92/01**

(84) Benannte Vertragsstaaten:
**CH DE FR GB IT LI**

(71) Anmelder: **BAYER AG**

**W-5090 Leverkusen 1 Bayerwerk(DE)**

(72) Erfinder: **Majer, Norbert, Dr.**
**Eibenweg 8**
**W-5060 Bergisch Gladbach 2(DE)**

(54) Verfahren zur Herstellung sehr reiner 5-Aminosalicylsäure.

(57) Sehr reine 5-Aminosalicylsäure, die als Ausgangsprodukt zur Herstellung von 5-Aminosalicyl-
säure in pharmazeutischer Qualität geeignet ist, wird
durch ein Verfahren erhalten bei dem Azobenzol-4-
hydroxy-3-carbonsäure mittels Alkali- oder Ammoniumhydrogensulfid und/oder -polysulfid unter erhöhtem Druck reduziert wird, das gebildete Anilin destillativ entfernt wird, und das dadurch gekennzeichnet
ist, daß die Reinigung des verbleibenden Rohproduktes ohne Zwischenisolierung durchgeführt wird
und vier aufeinanderfolgende Schritte umfaßt.

EP 0 463 466 A2

Rank Xerox (UK) Business Services

Die vorliegende Erfindung betrifft ein Verfahren zur Herstellung sehr reiner 5-Aminosalicylsäure.

5-Aminosalicylsäure hoher Reinheit wird zur Herstellung der pharmazeutischen Qualität benötigt. Für solche Zwecke geeignete 5-Aminosalicylsäure darf nur Verunreinigungen von max. 0,5 % Salicylsäure, max. 0,2 % 3-Aminosalicylsäure, max. 0,1 % 4-Aminophenol und max. 30 ppm Anilin enthalten. Die nach dem erfindungsgemäßen Verfahren hergestellte 5-Aminosalicylsäure erfüllt diese hohen Reinheitsansprüche. 5-Aminosalicylsäure in pharmazeutischer Qualität wird z.B. als Antiphlogistikum bei der Therapie von Morbus Crohn und Collitis Ulcerosa eingesetzt.

Aus Erfinderschein SU 1 159 919 ist die Reduktion von Azobenzol-4-hydroxy-3-carbonsäure mit Natriumhydrogensulfid bekannt. Nach der destillativen Abtrennung des Anilins erfolgt dort eine Zwischenisolierung des Produktes, welches durch Schwefel verunreinigt ist. Zur Entfernung des Schwefels wird erneut salzsauer gelöst, vom Schwefel abgetrennt und das Produkt durch Zusatz von Natronlauge wieder ausgefällt.

Diese Vorgehensweise führt zu Ausbeuteverlusten von über 10% verglichen mit dem erfindungsgemäßen Verfahren und zu einem unreineren Produkt.

Aus dem Stand der Technik sind weitere Verfahren zur Herstellung von 5-Aminosalicylsäure bekannt. Sie unterscheiden sich jedoch von dem erfindungsgemäßen Verfahren hinsichtlich der verwendeten Reduktionsmittel und in der Art der reinigenden Aufarbeitung (vgl. US 4,670,112; DD 255 941; DE 3 638 364).

In keinem der bekannten Verfahren wird jedoch eine so hohe Reinheit verbunden mit einer so hohen Ausbeute wie im erfindungsgemäßen Verfahren erzielt.

Es wurde nun ein Verfahren zur Herstellung sehr reiner 5-Aminosalicylsäure durch Reduktion von Azobenzol-4-hydroxy-3-carbonsäure mittels Alkali- oder Ammoniumhydrogensulfid und/oder -polysulfid unter erhöhtem Druck und destillativer Entfernung des gebildeten Anilins gefunden, das dadurch gekennzeichnet ist, daß die Reinigung des verbleibenden Rohproduktes ohne Zwischenisolierungen durchgeführt wird und folgende aufeinanderfolgende Schritte umfaßt:

a) Einstellung eines pH-Wertes von < 0.5

b) Entfernung von Schwefelwasserstoff und Schwefeldioxid

c) Entfernung von Schwefel und salzsäureunlöslichen Verunreinigungen

d) Ausfällung der 5-Aminosalicylsäure bei pH 2-4 und Isolierung

Das erfindungsgemäße Verfahren liefert 5-Aminosalicylsäure beispielsweise in über 99 prozentiger Reinheit mit Ausbeuten von beispielsweise 86-90 % der Theorie, bezogen auf Salicylsäure.

Die als Ausgangsprodukt für die Reduktion verwendete Azobenzol-4-hydroxy-3-carbonsäure kann in an sich bekannter Weise, beispielsweise durch Diazotierung von Anilin und Kupplung auf Salicylsäure hergestellt werden (vgl. H.E. Fierz-David, L. Blangey, Grundlegende Operationen der Farbenchemie, 8. Aufl., Springer-Verlag, Wien 1952, S. 150-151).

Für die Durchführung des erfindungsgemäßen Verfahrens hat es sich jedoch als vorteilhaft erwiesen, zur diazotierten Anilinlösung Natriumsalicylatlösung hinzuzudosieren, unter indirekter Kühlung sowie Einstellung eines pH-Wertes von 10-10,5 mit verdünnter Natronlauge, wodurch stets definierte Konzentrationsverhältnisse vorliegen.

Die Kupplungssuspension wird vorzugsweise ohne Zwischenisolierung der 5-Azobenzolsalicylsäure mit Natronlauge auf einen pH-Wert von 12 eingestellt und reduziert.

Bei der Durchführung des erfindungsgemäßen Verfahrens wird als Reduktionsmittel vorzugsweise Natrium-, Kalium- oder Ammoniumhydrogensulfid, insbesondere Natriumhydrogensulfid verwendet.

Das Reduktionsmittel wird beispielsweise in Mengen von 1,5 bis 2 Mol, vorzugsweise in Mengen von 1,6 bis 1,8 Mol bezogen auf 1 Mol Salicyclsäure eingesetzt.

Die Reduktion erfolgt beispielsweise bei einem Druck von 2 bis 4 bar, vorzugsweise 2 bis 3 bar.

Die Destillation zur Entfernung des Anilins kann beispielsweise mittels einer Füllkörper-Kolonne erfolgen, vorzugsweise arbeitet man nach dem Prinzip der Azeotrop-Destillation.

Die Reduktion des Kupplungsproduktes Azobenzol-4-hydroxy-3-carbonsäure sowie die anschließende Destillation und Reinigung gemäß den erfindungsgemäßen Schritten a) bis d) werden vorzugsweise ohne jede Zwischenisolierung von Reaktionsprodukten vorgenommen.

Die Reinigung des Rohproduktes erfolgt gemäß dem erfindungsgemäßen Verfahren in vier aufeinanderfolgenden Schritten, wobei

- In Schritt a) ein pH-Wert von vorzugsweise 0 bis 0,5 eingestellt wird. Zur Einstellung dieses pH-Wertes können beispielsweise Mineralsäuren verwendet werden. Vorzugsweise wird Salzsäure verwendet.

- In Schritt b) zur Entfernung von Schwefelwasserstoff und Schwefeldioxid das Reaktionsgemisch beispielsweise zunächst auf eine Temperatur zwischen 80 und 95 °C, vorzugsweise 85 und 90 °C erhitzt wird, und bei dieser Temperatur beispielsweise 2-10 m³/h, vorzugsweise 4-5 m³/h pro 10 m³ Reaktionsgemisch Inertgas durch das Reaktionsgemisch geblasen wird. Vorzugsweise wird als Inertgas Stickstoff verwendet.

- In Schritt c) zur Entfernung von Schwefel und salzsäureunlöslichen Verunreinigungen kann beispielsweise zunächst Schwefel zur Koagulation gebracht werden, indem bei einer Temperatur von beispielsweise 80 bis 95°C, vorzugsweise 85 bis 90°C beispielsweise 2-10 m³/h, vorzugsweise 4-5 m³/h Luft pro 10 m³ Reaktionsgemisch durch das Reaktionsgemisch geblasen werden, und anschließend unter Zusatz von beispielsweise 25-50 kg Aktivkohle pro 10 m³ Reaktionsgemisch unter Beibehaltung des als vorzugsweise angegebenen Temperaturbereichs von 85 bis 90°C filtriert wird. Dabei werden auch andere salzsäureunlösliche Verbindungen abgetrennt.

In Schritt d) zur Ausfällung der 5-Aminosalicylsäure beispielsweise das stark saure Reaktionsgemisch auf einen pH-Wert von vorzugsweise 2,2-2,6 eingestellt,die Temperatur von vorzugsweise 85 bis 90°C auf vorzugsweise 20 bis 25°C abgesenkt und die 5-Aminosalicylsäure beispielsweise durch Abnutschen oder Abfiltrieren isoliert wird.

Die Einstellung des pH-Bereiches erfolgt beispielsweise mit Alkali- oder Erdalkalihydroxiden, -carbonaten oder -hydrogencarbonaten, vorzugsweise verwendet man verdünnte Natronlauge von 5-20 Gew.-%.

Die Absenkung der Temperatur kann vor, gleichzeitig mit oder nach erfolgter Änderung des pH-Wertes erfolgen, vorzugsweise vor der Einstellung des pH-Wertes.

Dadurch, daß die erfindungsgemäße reinigende Aufarbeitung im sauren pH-Bereich durchgeführt wird, kann eine Oxidation der in dieser Hinsicht empfindlichen 5-Aminosalicylsäure vermieden werden.

Das erfindungsgemäße Verfahren wird ohne jede Zwischenisolierung weder von Azobenzol-4-hydroxy-3-carbonsäure noch von 5-Aminosalicylsäure durchgeführt, was eine Abwasserverringerung und Ausbeutesteigerung zur Folge hat.

Bei dem erfindungsgemäßen Verfahren mit seinen aufeinanderfolgenden Schritten der Reinigung tritt ein synergistischer Effekt auf, denn es werden gleichzeitig eine hohe Reinheit von über 98% und hohe Ausbeuten von über 86% erzielt.

Beispiel 1

In einem 8000 l fassenden emaillierten Rührwerksbehälter wurden 2500 l Wasser, 970 l wäßrige Salzsäure (30 gew.-%ig) und 360 kg Anilin (3,866 kmol) vorgelegt. Mit Sole wurde auf -10°C gekühlt und 870 l einer 27 gew.-%igen Natriumnitritlösung (3,866 kmol) so unter die Oberfläche eingeleitet, daß die Temperatur unter 0°C bleib und am Ende der Zugabe ein Kleiner Nitritüberschuß verblieb.

Dieser wurde mit 0,5 kg Amidosulfonsäure zerstört.

Innerhalb von 2,5 Stunden wurden bei 0 bis +5°C 2020 l einer 22 gew.-%igen Natriumsalicylatlösung (3,62 kmol) zugegeben und danach auf einen geringen Überschuß an diazotiertem Anilin geprüft. Bei 0 bis 8°C wurde in 3,5 Stunden durch Zugabe von 230 l Natronlauge (22 gew.-%ig) ein pH-Wert von 10,3 eingestellt.

Nach beendeter Kupplung wurde die Reaktionslösung bei Raumtemperatur in einem 12500 l fassenden druckfesten Stahl-Rührwerksbehälter überführt, 280 l Natronlauge (50 gew.-%ig) zugesetzt und mit Wasser auf 8800 l aufgefüllt. Es wurde auf 90-95°C aufgeheizt, 1200 kg Natriumhydrogensulfid-Lösung (31 gew.-%ig) zugegeben und der Rührwerksbehälter geschlossen.

Die Reaktionsmischung wurde auf 130°C geheizt und 3 Stunden bei dieser Temperatur gehalten. Dabei stellte sich ein Druck von 3 bar ein. Anschließend wurde auf 90°C gekühlt, der Rührwerksbehälter entspannt und das freigesetzte Anilin azeotrop abdestilliert, bis der Gehalt an Anilin kleiner als 0,1 % betrug und mit Wasser auf 9000 l aufgefüllt.

In einem 12 500 l fassenden emaillierten Rührwerksbehälter wurden 2150 l Salzsäure (30 gew.-%ig) vorgelegt und die Reaktionsmischung in 2 Stunden zudosiert, wobei eine Temperatur von 85°C erreicht wurde und sich ein pH-Wert von kleiner 0,5 einstellte. Dabei wurden Schwefelwasserstoff und Schwefeldioxid freigesetzt.

Der restliche Schwefelwasserstoff und das durch Zersetzung von Thiosulfat gebildete Schwefeldioxid wurde bei 85°C zunächst 2 Stunden lang mit Stickstoff, danach 15 Stunden mit Luft ausgeblasen, wodurch der freigesetzte Schwefel in einer gut filtrierbaren Form anfiel.

Das Reaktionsgemisch wurde auf Sulfidgehalt geprüft, mit 45 kg Aktivkohle versetzt, 0,5 Stunden bei 85°C nachgerührt, bei dieser Temperatur über ein Klärfilter geklärt und das Filtergut mit 100 l Heißwasser (90°C) nachgewaschen.

Das geklärte Filtrat wurde auf 30°C abgekühlt, langsam mit 1650 l Natronlauge (20 gew.-%ig) versetzt, wobei sich ein pH-Wert von 2,4 einstellte. Danach wurde auf 20 bis 25°C abgekühlt, filtriert, die isolierte 5-Aminosalicylsäure mit 2500 l Wasser chloridfrei gewaschen, der Filterkuchen trockengeblasen und die 5-Aminosalicylsäure isoliert.

Es wurden 860 kg feuchtes Produkt erhalten, entsprechend 505 kg getrocknetem Produkt.

Analyse: 99,1 % 5-Aminosalicylsäure (aus potentiometrischer Titration), < 0,1 % 3-Aminosalicylsäure (HPLC), 0,1 % Salicylsäure (HPLC), < 0,1 % 4-Aminophenol (HPLC) sowie < 10 ppm Anilin (DC).

Ausbeute: 90 % der Theorie, bezogen auf Salicylsäure.

Beispiel 2

Entsprechende Ergebnisse wurden erhalten, wenn man zu vorgelegtem Reaktionsgemisch Salzsäure hinzufügt und sonst so verfährt wie in Beispiel 1.

Beispiel 3

Entsprechende Ergebnisse wurden erhalten, wenn man den pH-Wert 2,4 des geklärten Filtrats bei 80-85°C einstellt, danach auf 20-25°C abkühlt und sonst so verfährt wie in Beispiel 1.

**Patentansprüche**

1. Verfahren zur Herstellung sehr reiner 5-Aminosalicylsäure durch Reduktion von Azobenzol-4-hydroxy-3-carbonsäure mittels Alkali- oder Ammoniumhydrogensulfid und/oder -polysulfid unter erhöhtem Druck und destillativer Entfernung des gebildeten Anilins, dadurch gekennzeichnet, daß die Reinigung des verbleibenden Rohproduktes ohne Zwischenisolierung erfolgt und folgende aufeinanderfolgende Schritte umfaßt:
   a) Einstellung eines pH-Wertes von < 0,5
   b) Entfernung von Schwefelwasserstoff und Schwefeldioxid
   c) Entfernung von Schwefel und salzsäureunlöslichen Verunreinigungen
   d) Ausfällung der 5-Aminosalicylsäure bei pH 2 bis 4 und Isolierung.

2. Verfahren nach Anspruch 1, dadurch gekennzeichnet, daß die Reduktion des Kupplungsproduktes Azobenzol-4-hydroxy-3-carbonsäure sowie die anschließende Destillation und Reinigung gemäß den erfindungsgemäßen Schritten a) bis d) ohne jede Zwischenisolierung von Reaktionsprodukten vorgenommen werden.

3. Verfahren nach Anspruch 1, dadurch gekennzeichnet, daß als Reduktionsmittel Natriumhydrogensulfid verwendet wird.

4. Verfahren nach Anspruch 1, dadurch gekennzeichnet, daß in Schritt a) ein pH-Wert von 0 bis 0,5 eingestellt wird.

5. Verfahren nach Anspruch 1, dadurch gekennzeichnet, daß in Schritt b) das Reaktionsgemisch auf eine Temperatur zwischen 85 und 90°C erhitzt wird, und bei dieser Temperatur Stickstoff durch das Reaktionsgemisch geblasen wird.

6. Verfahren nach Anspruch 1, dadurch gekennzeichnet, daß in Schritt c) bei einer Temperatur von 85 bis 90°C Luft durch das Reaktionsgemisch geblasen wird und anschließend unter Zusatz von Aktivkohle bei einer Temperatur zwischen 85 und 90°C filtriert wird.

7. Verfahren nach Anspruch 1, dadurch gekennzeichnet, daß in Schritt d) die Temperatur von 85 bis 90°C auf 20 bis 25°C abgesenkt, das stark saure Reaktionsgemisch auf einem pH-Wert von 2,2-2,6 eingestellt, und die 5-Aminosalicylsäure isoliert wird.

8. Verfahren nach Anspruch 1, dadurch gekennzeichnet, daß in Schritt d) die Einstellung des pH-Wertes mit verdünnter Natronlauge von 5-20 Gew.-% erfolgt.

9. Verwendung von gemäß Ansprüchen 1-7 hergestellter 5-Aminosalicylsäure als Ausgangsprodukt zur Herstellung von 5-Aminosalicylsäure in pharmazeutischer Qualität.